# EUROPÄISCHE PATENTSCHRIFT

(11) **EP 0 881 487 B1**
(45) Veröffentlichungstag und Bekanntmachung des Hinweises auf die Patenterteilung: **26.09.2007**
(21) Anmeldenummer: 98890164.1
(22) Anmeldetag: 28.05.1998
(51) Int. Cl.: G01N 31/22, G01N 33/84, C07D 413/10

(54) **Verfahren zur Bestimmung eines Alkaliions**
Method for the determination of an alkali ion
Méthode pour la détermination d'un ion alcalin

(30) Priorität: 30.05.1997 AT 93097
(43) Veröffentlichungstag der Anmeldung: 02.12.1998
(73) Patentinhaber: F. HOFFMANN-LA ROCHE AG, 4070 Basel (CH)
(72) Erfinder: Leiner, Marco Jean Pierre, 8045 Graz (AT); He, Huarui, Alpharetta, GA 30202 (US); Boila-Göckel, Andrei, 8010 Graz (AT)
(74) Vertreter: Schwarz, Albin

(56) Entgegenhaltungen:
- EP-A- 0 369 733
- BLACKBURN C ET AL: "Lithium responsive fluorophores derived from monoaza-12-crown-4 and coumarin. The influence of a methoxy side-arm on photophysical properties" TETRAHEDRON LETTERS, Bd. 35, Nr. 43, 24. Oktober 1994 (1994-10-24), Seiten 7915-8, XP002121089
- JONKER S A ET AL: "Cation complexation with functionalized 9-arylacridinium ions: possible applications in the development of cation-selective optical probes" RECUEIL DES TRAVAUX CHIMIQUES DES PAYS-BAS, Bd. 108, Nr. 3, März 1989 (1989-03), Seiten 109-15, XP002121090

## Beschreibung

Die vorliegende Erfindung betrifft ein Verfahren zur Bestimmung eines Alkaliions in einer Probe, wobei das Alkaliion mit einer Verbindung (=Luminophor-Ionophor), die einen luminophoren Rest und einen ionophoren Rest aufweist, in Kontakt gebracht wird, welcher ionophore Rest mit dem in der Probe enthaltenen Alkaliion reagiert, wobei der luminophore Rest seine Lumineszenzeigenschaften ändert, wonach die Lumineszenz gemessen und mit dem Meßergebnis auf die Konzentration oder die Aktivität des Alkaliions geschlossen wird, d.h. das Alkaliion bestimmt wird. Die Erfindung betrifft auch Monoaza-Kronenether, die als Luminophor-Ionophore zur Bestimmung eines Alkaliions eingesetzt werden können.

Ein derartiges Bestimmungsverfahren beruht auf dem sogenannten "PET-Effekt". Darunter wird ein mittels Photonen induzierter Elektronen-Transfer (photoinduced electron transfer = PET) vom ionophoren Rest bzw. Ionophor auf den luminophoren Rest bzw. Luminophor verstanden, der zu einer Verringerung der (relativen) Lumineszenzintensität und der Lumineszenzabklingzeit des Luminophors führt. Die Absorptions- und die Emissionswellenlängen werden jedoch dabei im wesentlichen nicht beeinflußt (J.R. Lakowicz in "Topics in Fluorescence Spectroscopy", Band 4: Probe Design and Chemical Sensing; Plenum Press, New York & London (1994)).

Durch die Bindung von Ionen an den Ionophor wird der PET-Effekt teilweise oder vollständig blockiert, sodaß es zu einem Anstieg der Lumineszenz des luminophoren Restes kommt. Somit kann durch Messung der Änderung der Lumineszenzeigenschaften, d.h. der Lumineszenzintensität und/oder der Lumineszenzabklingzeit, auf die Konzentration oder die Aktivität des zu bestimmenden Ions geschlossen werden.

Aus der US-A - 5,516,911 sind Fluoreszenzindikatoren für die intracelluläre Kalziumbestimmung bekannt, welche fluoreszierende Substituenten tragen, die als optische Indikatoren wirken können.

Ein Verfahren der eingangs beschriebenen Art ist aus der US-A - 5,439,828 bekannt, wobei als Luminophor-Ionophor Diaza-Kryptanden verwendet werden, die mit fluoreszierenden Cumarinen als Fluorophor funktionalisiert sind und je nach Struktur für Lithium-, Natrium- bzw. Kaliumionen spezifisch sind. Es wird angegeben, daß diese Luminophor-Ionophore in pH-neutralen Probemedien verwendet werden können und in solchen Systemen auch bevorzugt eingesetzt werden.

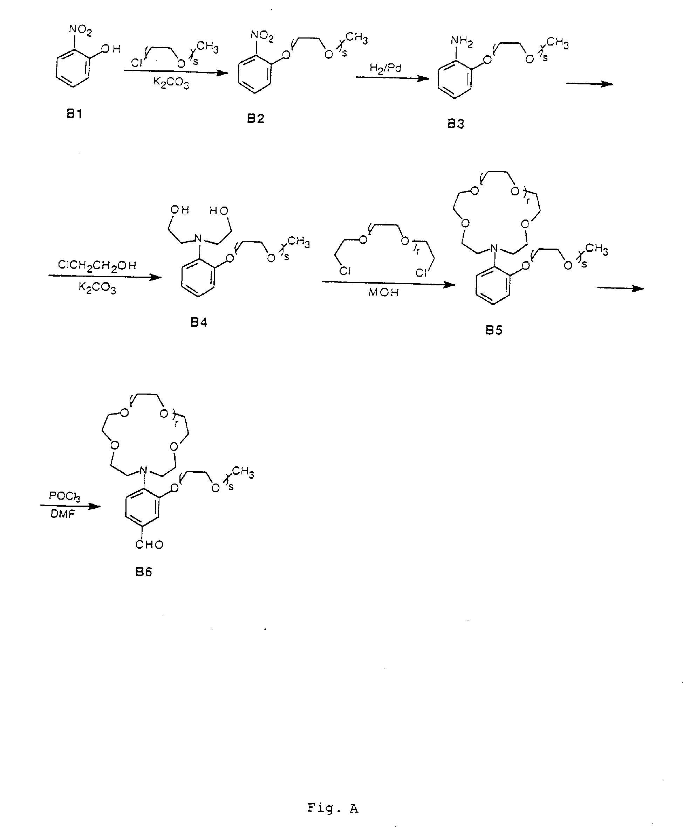

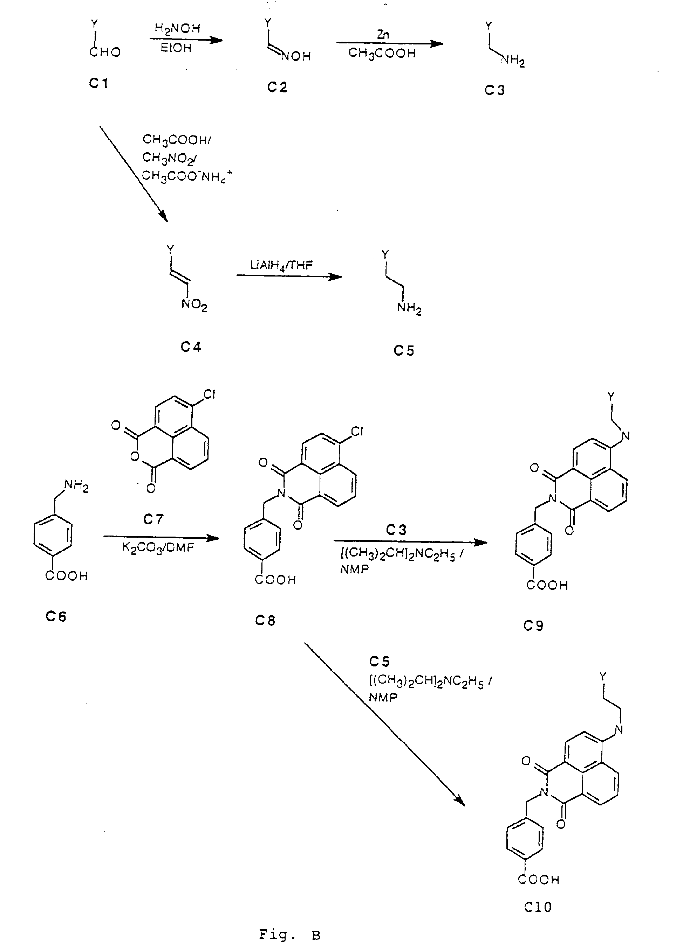

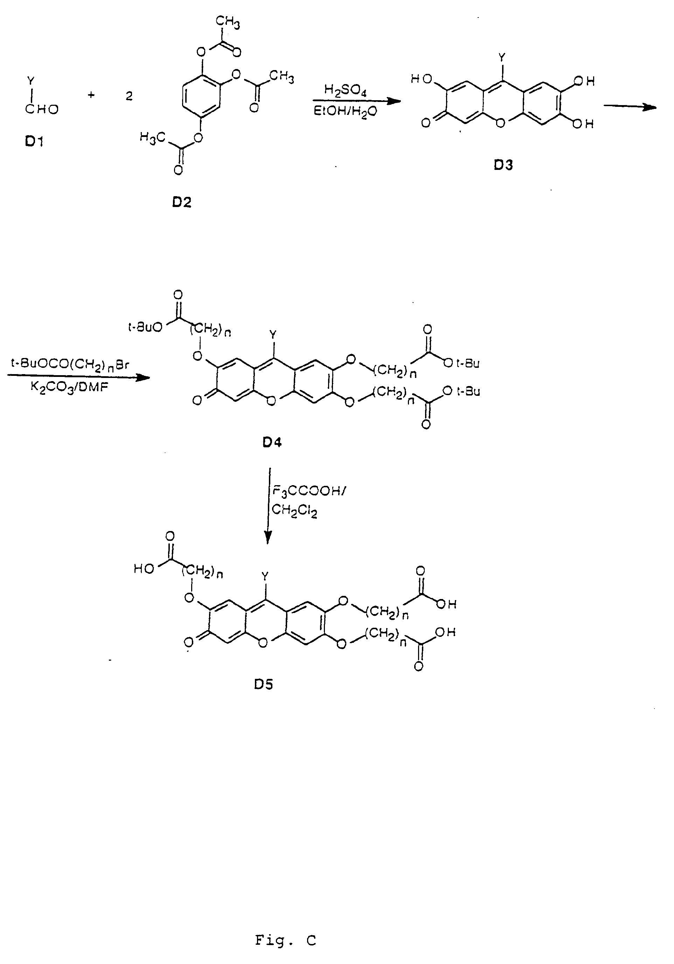

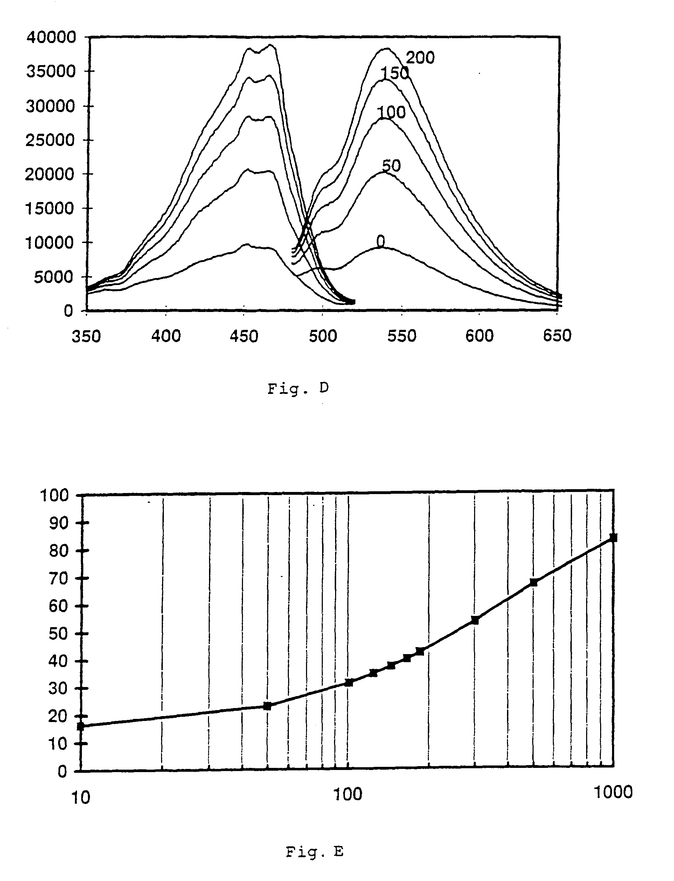

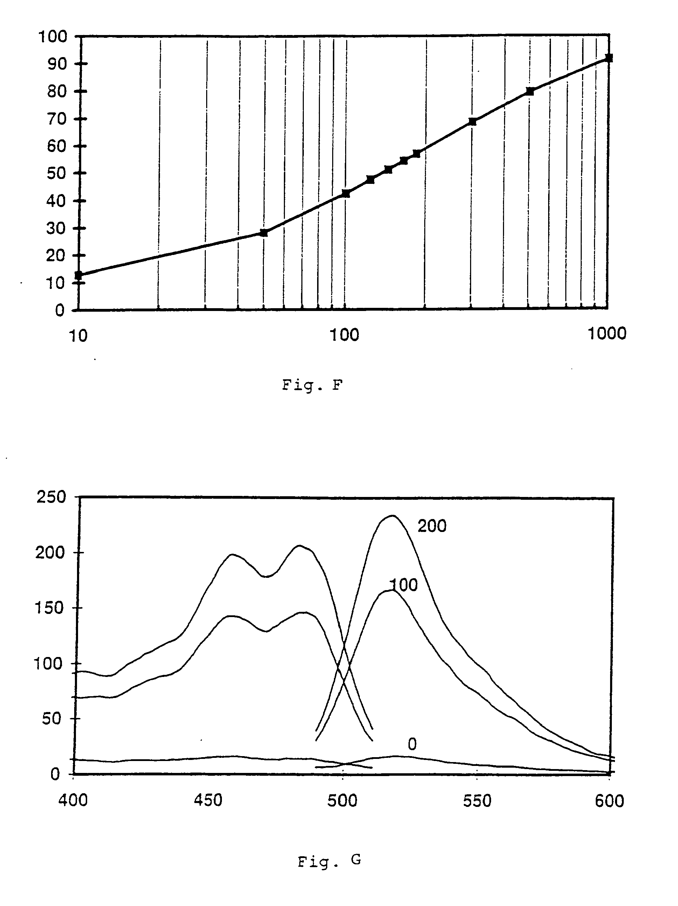

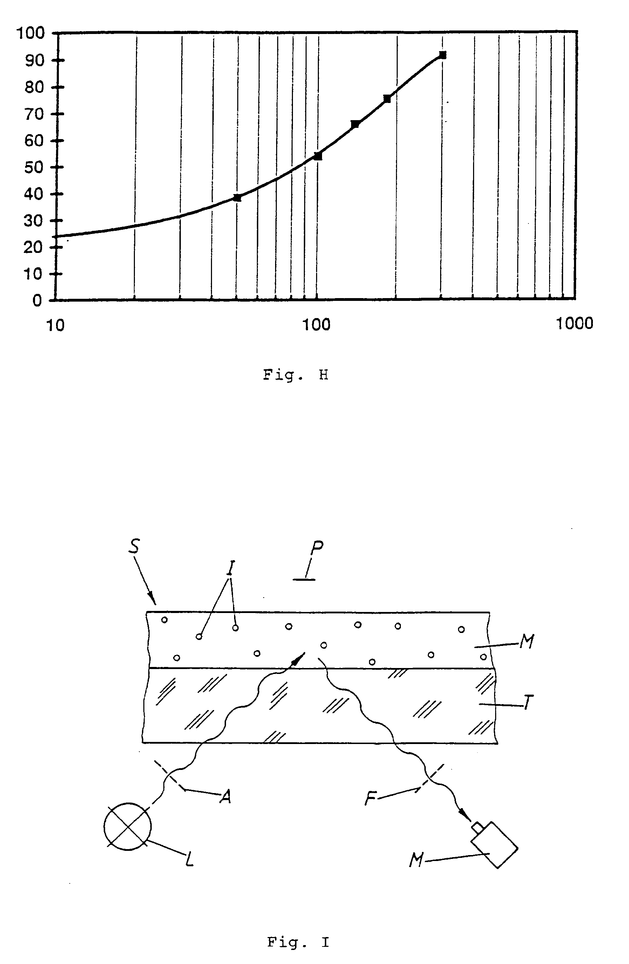

Untersuchungen (Frank Kastenholz, Inaugural-Dissertation, Universität zu Köln, 1993, Abb. 32, Seite 54) haben jedoch gezeigt, daß das Fluoreszenzsignal im physiologischen pH-Bereich signifikant vom pH-Wert der Probe abhängt und mit fallendem pH-Wert bereits ab einem pH von 7,4 stark ansteigt. Dies beeinträchtigt die Genauigkeit einer Bestimmung, die in biologischen Proben durchgeführt wird. Ferner haben die verwendeten Verbindungen den weiteren Nachteil, daß die verwendeten Cumarine Absorptionswellenlängen von etwa 336 nm aufweisen und daher nicht von kommerziellen LEDs angeregt werden können.

Diese Nachteile gelten auch für die in der US-A - 5,162,525 genannten Luminophor-Ionophore.

Aus Tetrahedron Letters, Band 31, Nr. 36, Seiten 5193-5196 (1990) sind Diaza-Kryptanden bekannt, bei denen beide Stickstoffatome an jeweils einen aromatischen Ring gebunden sind, d.h. Aryl-Stickstoffe bzw. Stickstoffe vom Anilintypus sind. Untersuchungen der Anmelderin haben gezeigt, daß sich diese Diaza-Kryptanden nicht zur Bestimmung von Kaliumionen eignen, wenn sie im physiologischen Konzentrationsbereich und bei physiologischen pH-Werten des Blutes (7,0-7,6) vorliegen.

Aus Tetrahedron Letters, Band 35, Nr.43, Seiten 7915-7918 (1994) ist ein System zur Bestimmung von Lithiumionen bekannt, bei dem ein Luminophor-substituierter Monoaza-Kronenether als aktive Komponente verwendet wird, wobei das Luminophor über einen Benzenring am Kronenether hängt. Der Benzenring ist dabei in der ortho-Position zum Kronenether mit einer Methoxyfunktion substituiert, die die freie Drehbarkeit des Kronenethers einschränkt. Hingegen ist der Benzenring mit dem Luminophor über Doppelbindungen konjugiert.

Die vorliegende Erfindung stellt sich daher die Aufgabe, das vorbekannte Verfahren so zu verbessern bzw. Luminophor-Ionophore zur Verfügung zu stellen, die bei physiologischen pH-Werten keine signifikante Abhängigkeit der Lumineszenzeigenschaften vom pH-Wert der Probe zeigen und daher zur Bestimmung bei biologischen Proben geeignet sind.

Das erfindungsgemäße Verfahren soll ferner insbesondere bei Vorliegen physiologischer Alkaliionenkonzentrationen gut durchführbar sein, d.h. eine große Abhängigkeit des Lumineszenzsignals von der Konzentration des zu bestimmenden Alkaliions aufweisen.

Diese Aufgabe wird bei dem eingangs beschriebenen Verfahren dadurch gelöst, daß als Verbindung (=Luminophor-Ionophor) ein Monoaza-Kronenether der allgemeinen Formel I 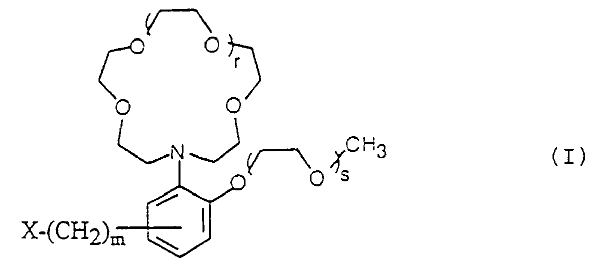 worin X der luminophore Rest ist, m die Zahl 0, 1 oder 2 ist, und r und s unabhängig voneinander die Zahlen 0, 1 oder 2 bedeuten, eingesetzt wird.

Die Monoaza-Kronenether mit der obigen allgemeinen Formel I sind neu. Es hat sich gezeigt, daß sich diese neuen Luminophor-Ionophore sehr gut zur Bestimmung von Alkaliionen bei physiologischen pH-Werten und bei physiologischen Konzentrationen eignen.

Die erfindungsgemäßen Monoaza-Kronenether eignen sich insbesondere gut zur Bestimmung von Natriumionen im Konzentrationsbereich zwischen 110 und 180 mmol/l.

Ohne an eine bestimmte Theorie gebunden zu sein, wird angenommen, daß bei den erfindungsgemäßen Monoaza-Kronenethern die vorteilhaften Eigenschaften darauf zurückzuführen sind, daß der Stickstoff ein aromatischer Stickstoff ist.

Als luminophorer Rest X eignen sich alle Reste, mit welchen sich in Kombination mit dem ionophoren Rest ein PET-Effekt erzielen läßt. Aus der Literatur ist eine Vielzahl von Resten bekannt, die in Kombination mit dem Ionophor den PET-Effekt ergeben oder sich prinzipiell dafür eignen. Durch Ankoppelung dieser vorbekannten Reste an den Benzolring der allgemeinen Formeln I und II werden neue Verbindungen erhalten, an denen der Fachmann prüfen kann, ob sich ein PET-Effekt erzielen läßt. Die Ankoppelung kann zum Stickstoff in ortho-Position, in seinen beiden meta-Positionen und in para-Position erfolgen. Bevorzugt ist die para-Position.

Wie dem Fachmann bekannt ist, ist für das Zustandekommen eines PET-Effektes insbesondere eine elektronische Entkoppelung des Elektronendonors des ionophoren Restes vom elektronischen System des luminophoren Restes wesentlich. Diese elektronische Entkoppelung von ionophorem und luminophorem Rest kann bekanntermaßen dadurch erreicht werden, daß beide Reste entweder durch eine Spacer-Gruppe, also die (CH₂)ₘ-Kette mit m>0 oder - wenn m=0 - durch einen virtuellen Spacer (z.B. durch Verdrehung der Ebene des luminophoren Restes zur Ebene des Benzolringes) getrennt vorliegen. Die Funktion des Spacers besteht somit darin, einer Konjugation des Elektronensystems des ionophoren Restes mit dem Elektronensystem des luminophoren Restes entgegenzuwirken.

Die elektronische Entkoppelung ist z.B. daran erkenntlich, daß sich die Absorptions- und Emissionsspektren hinsichtlich ihrer Wellenlänge nicht wesentlich ändern.

Zur Bestimmung von Natriumionen wird bevorzugt ein Monoaza-Kronenether der allgemeinen Formel I eingesetzt, in welcher r und s die Zahlen 1 bzw. 0 bedeuten.

Zur Bestimmung von Kaliumionen wird ferner bevorzugt ein Monoaza-Kronenether der allgemeinen Formel I eingesetzt, in welcher r und s die Zahlen 2 bzw. 1 bedeuten.

Der luminophore Rest X in der allgemeinen Formel I ist bevorzugt
- ein Amino-Naphthalimid-Rest der allgemeinen Formel II 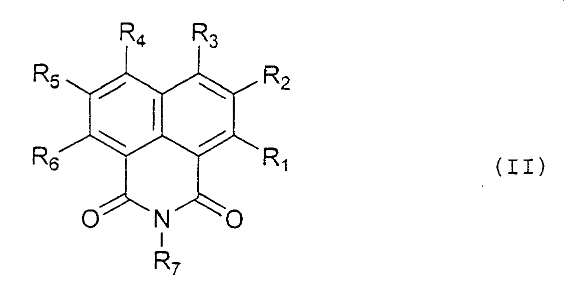 in welcher eine von R₁, R₂, R₃, R₄, R₅ und R₆ eine Gruppe -NH- ist, über welche X an die Gruppe -(CH₂)ₘ- der oben genannten Verbindung mit der allgemeinen Formel I gebunden ist, und die anderen und R₇ jeweils voneinander unabhängig Wasserstoff, eine lipophile oder hydrophile Gruppe oder eine reaktive Gruppe zur Kopplung an ein Polymer sind, oder
- ein Xanthenon-Rest der allgemeinen Formel III 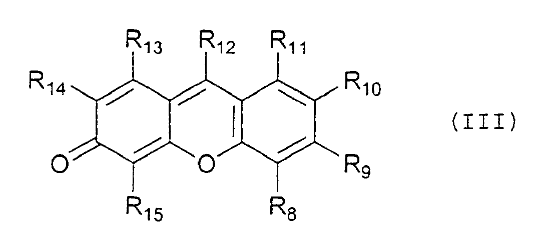 ist, in welcher eine von R₈, R₉, R_{10,} R₁₁, R₁₂, R₁₃, R₁₄ und R₁₅ für eine chemische Bindung steht, über welche X direkt (m=0) an den ionophoren Rest der oben genannten Verbindung mit der allgemeinen Formel I gebunden ist, und die übrigen für -OH, -OR₁₆, worin R₁₆ eine hydrophile oder eine lipophile Gruppe ist, -O-R₁₇-G, worin R₁₇ eine hydrophile oder eine lipophile Gruppe und G eine reaktive Gruppe zur Kopplung an ein Polymer ist, oder -(CH₂)ₙ-COOH, worin n eine ganze Zahl zwischen 0 und 17 ist, stehen.

Bevorzugt ist in der allgemeinen Formel II R₃ oder R₄ die Gruppe -NH-, über welche der luminophore Rest an die Gruppe -(CH₂)ₘ-der oben genannten allgemeinen Formel I gebunden ist.

Bevorzugt ist weiters in der allgemeinen Formel III R₁₂ eine chemische Bindung, über welche der luminophore Rest direkt (m=0) an den ionophoren Rest der oben genannten allgemeinen Formel I gebunden ist.

Als lipophile Gruppe eignen sich z.B. substituierte und unsubstituierte Alkylgruppen und Alkoxygruppen mit bis zu 20 C-Atomen.

Als hydrophile Gruppe eignen sich z.B. Alkylgruppen mit 1-17 C-Atomen, die zumindest eine Hydroxylgruppe tragen und/oder funktionelle Gruppen, die beim pH der Meßlösung in dissoziiertem Zustand vorliegen, wie z.B. Carbonsäuren, Sulfonsäuren und Phorphorsäuren.

Reaktive Gruppen zur Kopplung an aminofunktionalisierte Polymere, z.B. Aminocellulose und aminofunktionelle Polyacrylamide, sind z.B. aus der US-A - 4,774,339, Tabelle 4, bekannt.

Diese oben genannten, bevorzugt eingesetzten luminophoren Reste können mit Licht einer Wellenlänge von > 450 nm angeregt werden.

Die erfindungsgemäßen Verbindungen zur Bestimmung der Alkaliionen können in gelöster Form der Probelösung zugegeben werden. Sie können aber auch Bestandteil eines Sensors sein, in welchem sie in einer Schicht aus z.B. einem Hydrogel eingebettet vorliegen, wie unten an Hand der Figur I beschrieben wird.

Die Erfindung betrifft ferner einen Monoaza-Kronenether der allgemeinen Formel I 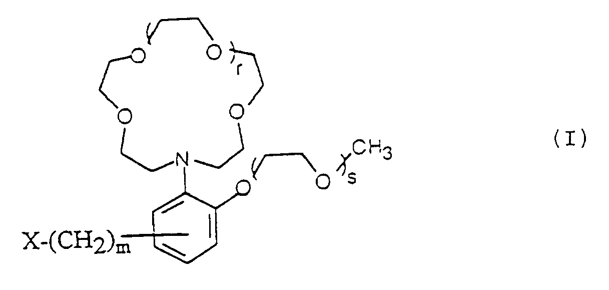 worin X der luminophore Rest ist und insbesondere die oben angegebene Bedeutung besitzt, m die ganze Zahl 0, 1 oder 2 ist, und r und s unabhängig voneinander die ganzen Zahlen 0, 1 oder 2 bedeuten.

Nachfolgend wird die Erfindung beispielhaft noch näher beschrieben, wobei Synthese und Eigenschaften einiger bevorzugt verwendeter Monoaza-Kronenether erläutert werden. Andere erfindungsgemäße Verbindungen können vom Fachmann auf analoge Weise hergestellt werden.

### 1. Synthese der erfindungsgemäßen Monoaza-Kronenether

### 1.1. Synthese des ionophoren Restes der erfindungsgemäßen Monoaza-Kronenether (Figur A)

Der Syntheseweg für den ionophoren Teil der erfindungsgemäßen Monoaza-Kronenether ist in der Figur A allgemein dargestellt.

Allgemeines Verfahren (Figur A)

Die Synthese von Monoaza-kronen-(Lariat)-ethern mit Seitenarmen wurde über zwei Hauptschritte vorgenommen: einer Alkylierung und einer Cyclisierung. 2-Nitrophenol B1 konnte in Dimethylformamid in Gegenwart von K₂CO₃ mit Chlorethyl-alkoxyethern mit verschiedener Kettenlänge (s=0, 1, 2) alkyliert werden. Die erhaltenen Nitroverbindungen B2 wurden zu Aminen B3 hydriert, gefolgt von einer Alkylierung der Aminogruppe in Chlorethanol mit K₂CO₃ als Base zu den 2-[N,N-bis(2-Hydroxyethylaminophenylalkoxyethyl-ethern B4 (s=0, 1, 2). Diese Bis-Hydroxy-Verbindungen B4 wurden mit Ethylglycol-dichlorethyl-ethern (r=0,1,2) in Dioxan mit Alkalihydroxid zu den Lariat-ethern B5 (r=0, 1, 2; s=0, 1, 2) cyclisiert. Diese Ether B5 (Phenylaza-kronenether) wurden formyliert, um die Zwischenprodukte B6 (r=0, 1, 2; s=0, 1, 2) zu erhalten.

Beschreibung einzelner Reaktionschritte der Figur A

N,N-Bis(2-hydroxyethyl)-2-methoxyanilin B4 (s=0) :

452 g (4 mol) o-Anisidin wurden in 1932 g (24 mol) 2-Chlorethanol gelöst und 15 min auf 80°C erhitzt. Dann wurden 608 g (4,4 mol) K₂CO₃ langsam zugegeben, um die Temperatur unter 110°C zu halten (exotherme Reaktion). Das Gemisch wurde 22 Stunden auf 95°C erhitzt und abgekühlt. Etwa 800 ml nicht umgesetztes Chlorethanol wurden abgedampft, und der Rückstand wurde mit 1 1 Wasser verdünnt und 2 mal mit 1 1 Chloroform extrahiert. Der Extrakt wurde 5 mal mit 1,5 1 Wasser gewaschen und über K₂CO₃ getrocknet. Das Lösungsmittel wurde abgedampft, wobei 404 g (Ausbeute: 48%) hellbraunes Öl erhalten wurden. Das Dünnschichtchromatogramm zeigte eine Reinheit von etwa 95%.
¹H-NMR (CDCl₃), δ (ppm): 3,18 (t, 4H), 3,50 (t, 4H), 3,60 (m, 2H), 3,82 (s, 3H), 6,90 (m, 2H), 7,10 (m, 1H), 7,19 (m, 1H).

2-Methoxyphenylaza-15-krone-5 B5 (s=0, r=1):

Dieser Schritt wurde in Anlehnung an J.P.Dix und F. Vögtle, Chem. Ber. 113, 457-470 (1980) vorgenommen.

403 g (1,91 mol) B4 (s=0) wurden in 2210 ml Dioxan gelöst und 20 min auf 80°C erhitzt. Dann wurden 168 g (4,20 mol) gemahlenes NaOH langsam innerhalb von 3 Stunden zugegeben. Die Temperatur wurde auf 95°C angehoben, als 300 ml (1,93 mol) Bis(2-chlorethoxyethan) in einer Portion zugegeben wurden, dann wurde das Reaktionsgemisch 30 Stunden auf 95°C erhitzt. Nach Filtration des heißen Gemisches wurde das Lösungsmittel abgedampft. Der Rückstand wurde mit einer Lösung von 234 g (1,91 mol) NaClO₄ in 640 ml Methanol behandelt. Das Gemisch wurde 30 min bei 60°C gerührt und auf etwa 300 ml konzentriert. 860 ml Ethylacetat wurden zugegeben, und es wurde anschließend 20 min bei Raumtemperatur gerührt. Dann wurde das Gemisch 2 Stunden bei Raumtemperatur stehengelassen.

Das erhaltene Präzipitat wurde filtriert, 2 mal mit 200 ml Ethylacetat gewaschen und 30 min bei Raumtemperatur getrocknet, wobei 199 g Azakrone-Natriumperchlorat-Komplex als weißes, weiches Pulver erhalten wurden. Dieses Pulver wurde in einem Gemisch aus 600 ml Dichlormethan und 600 ml Wasser gelöst, und die wässerige Phase wurde neuerlich mit 400 ml Dichlormethan extrahiert. Die organischen Schichten wurden vereinigt, 8 mal mit 600 ml entionisiertem Wasser gewaschen und über Na₂SO₄ getrocknet. Das Dichlormethan wurde abgedampft, wobei 100,4 g hellgelbes Öl (Ausbeute: 16%) erhalten wurden.
¹H-NMR (CDCl₃), δ (ppm) : 3,49 (t, 4H), 3,68 (t, 16H), 3,82 (s, 3H), 6,88 (m, 3H), 7,12 (m, 1H).

4-Formyl-2-methoxyphenylaza-15-krone-5 B6 (s=0, r=1):

100 g (308 mml) B5 (s=0, r=1) wurden in 145 ml (1850 mmol) Dimethylformamid in einem 500 ml Dreihalskolben gelöst und auf -5°C abgekühlt. 57,4 ml (616 mmol) POCl₃ wurden in einem Zugabetrichter tropfenweise zugegeben. Die Innentemperatur des Kolbens wurde 5°C nicht überschreiten gelassen. Dann wurde 16 Stunden bei Raumtemperatur gerührt, auf 500 g Eis gegossen und mit gesättigter wässeriger K₂CO₃-Lösung auf pH 7 gebracht. Die Lösung wurde 2 mal mit 500 ml Chloroform extrahiert. Die Chloroform-Phase wurde 2 mal mit 500 ml Wasser gewaschen, über 100 g MgSO₄ 1 Stunde getrocknet. Das Lösungsmittel wurde abgedampft, wobei 85 g hellgelbes Öl erhalten wurden, welches beim Stehenlassen über Nacht bei Raumtemperatur kristallisierte. Umkristallisation aus Ethylacetat/Hexan (1:4) ergab 56 g hellorange Kristalle (Ausbeute: 51%).
¹H-NMR (CDCl₃), δ (ppm): 3,68 (t, 16H), 3,78 (t, 4H), 3,82 (s, 3H), 7,05 (m, 1H), 7,28 (m, 2H), 9,78 (s, 1H).

### 1.2 Herstellung der erfindungsgemäßen Monoaza-Kronenether (Figuren B und C)

Die Herstellung der erfindungsgemäßen Monoaza-Kronenether ist in den Figuren B und C für zwei luminophore Reste (Amino-Naphthalimid bzw. Xanthenon) beispielhaft dargestellt, wobei "Y" jeweils für den ionophoren Rest steht.

Allgemeines Verfahren der Figur B (der luminophore Rest ist ein Amino-Naphthalimid-Rest)

Erfindungsgemäße Verbindungen mit einer einzigen CH₂-Gruppe als Spacer zwischen dem luminophoren und dem ionophoren Rest wurden hergestellt, indem zunächst die Verbindung C1, welche beispielsweise die Verbindung B6 (Fig. A) sein kann, in welcher "Y" für B5 steht, in das Oxim C2 übergeführt und mittels Zn in Essigsäure zum Amin C3 reduziert wurde. Die Kopplung an den luminophoren Rest zur Herstellung der erfindungsgemäßen Verbindung C9 ist in der Figur B an Hand des Amino-Naphthalimids C8, welches durch Umsetzung von C6 mit C7 in Dimethylformamid in Gegenwart von K₂CO₃ hergestellt wurde, schematisch gezeigt.

Erfindungsgemäße Verbindungen mit zwei CH₂-Gruppen als Spacer zwischen dem luminophoren und dem ionophoren Rest wurden hergestellt, indem zunächst die Verbindung C1, mit einem großen Überschuß an Nitromethan in der Gegenwart von Ammoniumacetat zur Verbindung C4 umgesetzt wurde, die anschließend mittels LiAlH₄ in THF zum Amin C5 reduziert wurde. Die Kopplung an das Amino-Naphthalimid C8 zur Herstellung der erfindungsgemäßen Verbindung C10 ist in der Figur B ebenfalls schematisch gezeigt.

Beschreibung einzelner Reaktionschritte der Figur B

4-Oximyl-2-methoxyphenylaza-15-krone-5 C2 (s=0, r=1):

Einer Lösung von 80 g (226 mmol) B6 (s=0, r=1) in 550 ml Ethanol wurde eine Lösung von 20,4 g (293 mmol) Hydroxylamin-hydrochlorid und 20,4 g (146 mmol) K₂CO₃ in 550 ml Wasser zugegeben. Das Gemisch wurde 16 Stunden bei Raumtemperatur und 3 Stunden bei 70°C gerührt. Dann wurde Ethanol abgedampft und der Rückstand in einem Gemisch von 500 ml Chloroform und 300 ml Wasser gelöst. Die wässerige Schicht wurde mit 500 ml Chloroform extrahiert. Die Chloroform-Extrakte wurden vereinigt und 2 mal mit 500 ml Wasser gewaschen und über K₂SO₄ getrocknet. Das Lösungsmittel wurde abgedampft, wobei 80,1 g gelbes Öl erhalten wurden (Ausbeute: 96%).

4-Aminomethyl-2-methoxyphenylaza-15-krone-5 C3 (s=0, r=1):

Einer Lösung von 45 g (122 mmol) C2 (s=0, r=1) in 450 ml Essigsäure wurden 78 g (1180 mmol) Zinkstaub langsam unter Eiswasserkühlung zugegeben. Diese Suspension wurde 18 Stunden bei Raumtemperatur und 3 Stunden bei 70°C gerührt, filtriert und 3 mal mit 200 ml Ethanol gewaschen. Das Lösungsmittel wurde abgedampft und das erhaltene Öl wurde in einem Gemisch aus 300 ml Chloroform und 300 ml Wasser gelöst und mit 6 n KOH auf pH 12 gebracht. Die wässerige Schicht wurde mit 500 ml Chloroform gewaschen. Die vereinigten Chloroform-Extrakte wurden 2 mal mit 500 ml Wasser gewaschen und über K₂CO₃ getrocknet. Das Lösungsmittel wurde abgedampft, wobei 35,2 g bräunlich-gelbes Öl (Ausbeute: 78%) erhalten wurden.

Das Dünnschichtchromatogramm zeigte eine Reinheit von etwa 70%, und der Fleck wurde bei Behandlung mit Ninhydrin bläulich-violett. Dieses Amin wurde ohne weitere Reinigung direkt im nächsten Schritt eingesetzt.

4-Nitroethylenyl-2-methoxyphenylaza-15-krone-5 C4 (s=0, r=1):

18,8 g (50 mmol) B6 (s=0, r=1), 38,5 g (500 mmol) Ammoniumacetat wurden in 100 ml Essigsäure suspendiert und 10 min bei Raumtemperatur gerührt. Dann wurden 59,4 ml (1100 mmol) Nitromethan zugegeben. Das Gemisch wurde 5 Stunden auf 60°C erhitzt und dann in Eiswasser gegossen. Die erhaltenen Kristalle wurden abfiltriert, mit Wasser gewaschen und in einem Exsikkator mit P₂O₅ getrocknet, wobei 11,9 g dunkelrote Nadeln (Ausbeute: 60%) erhalten wurden.
¹H-NMR (CDCl₃), δ (ppm): 3,68 (t, 16H), 3,78 (t, 4H), 3,82 (s, 3H), 6,88 (m, 1H), 7,08 (m, 2H), 7,45 (d, 1H), 7,85 (d, 2H).

4-Aminoethyl-2-methoxyphenylaza-15-krone-5 C5 (s=0, r=1):

Zu 200 ml Tetrahydrofuran in einem 500 ml Dreihalskolben wurden 3,8 g (100 mmol) LiAlH₄ langsam zugegeben. Dann wurden 4,0 g (10 mmol) C4 (s=0, r=1) in 50 ml Tetrahydrofuran innerhalb 2 Stunden tropfenweise zugegeben. Das Gemisch wurde 4 Stunden unter Rückfluß erhitzt und dann in einem Eisbad abgekühlt. 6 n KOH wurde zugegeben, um nicht umgesetztes LiAlH₄ zu zerstören. Dann wurde filtriert, das Lösungsmittel abgedampft und der Rückstand in 150 ml Chloroform gelöst, 2 mal mit 150 ml Wasser gewaschen, über 15 g K₂CO₃ getrocknet und das Lösungsmittel abgedampft, wobei 4,9 g oranges Öl (Ausbeute: 125%) erhalten wurden.

Das Dünnschichtchromatogramm zeigte eine Reinheit von etwa 80%, und der Fleck wurde unter Behandlung mit Ninhydrin bläulich-violett. Dieses Amin wurde ohne weitere Reinigung direkt weiterverwendet.

4-Chlor-N-(4-carboxyphenylmethyl)-1,8-naphthalimid C8:

46,4 g (200 mmol) 4-Chlor-1,8-naphthalinsäureanhydrid C7, 30,2 g (200 mmol) 4-Aminomethylbenzoesäure C6 und 13,8 g (100 mmol) K₂CO₃ wurden in 2 l Dimethylformamid suspensdiert, bei Raumtemperatur 16 Stunden und bei 60°C 6 Stunden gerührt. Das Gemisch wurde anschließend in 4 l Wasser gegossen und mit 6 n HCl auf pH 4 gebracht. Das erhaltene Präzipitat wurde abfiltriert und 18 min bei 60°C getrocknet, wobei 36 g schmutzig weißes Pulver erhalten wurden (Ausbeute: 51%).

4-{4'-[4''-C-(Aza-15-krone-5)-3"-methoxyphenylmethylamino]-1',-8'-naphthalimidylmethyl}-benzoesäure C9:

3,68 g (10 mmol) C3, 5,05 g (70%, 10 mmol) C8 (r=1, s=0) und 3,25 g (25 mmol) Diisopropylethylamin wurden in 25 ml N-Methylpyrrolidinon suspensdiert und 15 Stunden auf 110°C erhitzt, danach abgekühlt und in 475 ml 2%-ige wässerige Essigsäure gegossen. Das erhaltene Präzipitat wurde abfiltriert und 2 mal mit 100 ml Wasser gewaschen, in einem Exsikkator mit P₂O₅ 18 Stunden getrocknet, wobei 4,6 g braungelber Feststoff erhalten wurden, der ein Gemisch aus C3 und dem Produkt war.

Dieser Feststoff wurde in einem heißem Gemisch von Chloroform/Methanol (3:1) gelöst. Anschließend wurde filtriert. Das Filtrat wurde einer Säule aufgegeben, die mit 60 g Silikagel 100 gepackt war, es wurde mit Chloroform/Methanol (3:1) gespült, um nicht umgesetztes C3 zu entfernen, und dann wurde mit Chloroform/Methanol (3:1), enthaltend 1% Essigsäure, gespült, um 0,58 g (Ausbeute: 8,5%) des gewünschten Produktes zu erhalten.
¹H-NMR (D₃CS(=O)CD₃), δ (ppm): 3,25 (t, 4H), 3,50 (t, 16H), 3,75 (s, 3H), 4,15 (t, 1H), 4,58 (d, 2H), 5,25 (s, 2H), 6,78 (d, 1H), 6,88 (m, 2H), 7,38 (d, 2H), 7,55 (m, 2H), 7,82 (d, 2H), 8,20 (d, 2H), 8,50 (m, 1H), 8,80 (d, 1H).

C₄₂H₄₉N₃O₁₃ Berechnet für Diacetat: C 62,75; H 6,14; N 5,23.
Gefunden: C 62,37; H 6,09; N 5,12.

4-{4'- [4"-C-(Aza-15-krone-5)-3"-methoxyphenylethylamino]-1',-8'-naphthalimidylmethyl}-benzoesäure C10:

1,85 g (5 mmol) C5, 2,62 g (80%, 10 mmol) C8 (r=1, s=0) und 1,63 g (12,5 mmol) Diisopropylethylamin wurden in 12,5 ml N-Methylpyrrolidinon suspendiert und 15 Stunden auf 110°C erhitzt, anschließend wurde abgekühlt und in 238 ml wässerige 2%ige Essigsäure gegossen. Das erhaltene Präzipitat wurde abfiltriert und 2 mal mit 50 ml Wasser gewaschen, in einem Exsikkator mit P₂O₅ 18 Stunden getrocknet, wobei 1,8 g braungelber Feststoff erhalten wurden, der ein Gemisch aus C5 und dem Produkt war.

Dieser Feststoff wurde in 100 ml eines heißen Gemisches von Chloroform/Methanol (9:1) gelöst. Anschließend wurde filtriert. Das Filtrat wurde einer Säule aufgegeben, die mit 180 g Silikagel 100 gepackt war, es wurde mit Chloroform/Methanol (9:1) gespült, um nicht umgesetztes C5 zu entfernen, und dann wurde mit Chloroform/Methanol (3:1), enthaltend 1% Essigsäure, gespült, um 0,52 g (14,9%) des gewünschten Produktes zu erhalten.
¹H-NMR (D₃CS(=O)CD₃), δ (ppm): 2,90 (t, 2H), 3,25 (t, 4H), 3,50 (t, 16H), 3,60 (t, 2H), 3,75 (s, 3H), 4,45 (t, 1H), 5,25 (s, 2H), 6,78 (d, 1H), 6,88 (d, 1H), 6,95 (d, 1H), 7,25 (m, 2H), 7,65 (d, 1H), 7,80 (d, 2H), 7,95 (t, 1H), 8,25 (d, 1H), 8,45 (d, 1H), 8,75 (d, 1H).

FABMS (70 eV, m-Nitrobenzylalkohol-Dispersion mit LiJ): 711 (15%), (M+2Li-H); 670 (31%), (M-CO₂-H+2Li); 313 (100%) (Phenylazakrone+Li).

Verfahren der Figur C (der luminophore Rest ist ein Xanthenon-Rest)

8-[4'-C-(Aza-15-krone-5)-3'-methoxyphenyl]-2,3,7-trihydroxyfluor-6-one D3 (r=1, s=0) :

Einer Suspension von 6,51 g (25 mmol) Triacetoxybenzol in 60 ml 50% (v/v) Ethanol wurden 5 ml H₂SO₄ konz. tropfenweise zugegeben, 10 min gerührt, und dann wurden 4,42 g (12,5 mmol) B6 (s=0, r=1) in einer Portion zugegeben. Die erhaltene Suspension wurde 22 Stunden auf 80°C erhitzt. Das Ethanol war danach größtenteils abgedampft. Der Rückstand wurde mit 50 ml Wasser verdünnt, mit 25%-igem Tetramethylammoniumhydroxid auf pH 5 eingestellt und über Nacht stehengelassen. Die Flüssigkeit wurde dekantiert und der rote Feststoff wurde 3 mal mit 50 ml Wasser gewaschen und 3 mal mit 50 ml Methanol trituriert. Das Lösungsmittel wurde abgedampft, wobei 4,2 g (33,5%) roter Schaum, der direkt im nächsten Schritt eingesetzt wurde, erhalten wurden.

8-[4'-C-(Aza-15-krone-5)-3'-methoxyphenyl]-2,3,7-tri-t-butoxycarbonylmethylfluor-6-one D4 (r=1, s=0) :

1,14 (2 mmol) D3 mit r=1 und s=0, 1,00 g (6 mmol) KJ, 1,76 g (9 mmol) K₂CO₃ und 1,76 g (9 mmol) t-Butylbromacetat wurden in 10 ml Dimethylformamid suspendiert und 1 Stunde auf 110°C erhitzt. Das Gemisch wurde abgekühlt und mit 90 ml Wasser verdünnt. Diese Lösung wurde 2 mal mit 80 ml Chloroform extrahiert, 3 mal mit 160 ml Wasser gewaschen und über K₂CO₃ getrocknet. Das Lösungsmittel wurde abgedampft, wobei 1,50 g dunkelrote, gummiartige Substanz erhalten wurden. Dieses Rohprodukt wurde mit Silikagel 100 mit Chloroform/Methanol (9:1) gereinigt, wobei 0,95 g rotes Öl (Ausbeute: 59%) erhalten wurden.

8-[4'-C-(Aza-15-krone-5)-3'-methoxyphenyl]-2,3,7-tricarboxymethylfluor-6-one D5:

0,93 g (1,1 mmol) D3 (r=1, s=0) wurden in 2,5 ml Dichlormethan gelöst, und 0,5 ml Trifluoressigsäure (TFA) wurden zugegeben. Das Gemisch wurde 4 Stunden auf 40°C erwärmt. Dann wurden das Lösungsmittel und TFA abgedampft, und der Rückstand wurde in 10 ml Methanol gelöst, wonach das Methanol abgedampft wurde. Dieses Verfahren wurde 3 mal wiederholt, um die TFA vollständig zu entfernen, wobei 0,87 g rote, gummiartige Substanz erhalten wurden. Diese gummiartige Substanz wurde direkt für die unten beschriebene Immobilisierung verwendet.

### 2. Lumineszenzeigenschaften der erfindungsgemäßen Monoaza-Kronenether

Die Figuren D bis H zeigen die Lumineszenzeigenschaften von erfindungsgemäßen Verbindungen in Lösung und immobilisiert auf Cellulose in Abhängigkeit von der jeweiligen Konzentration an Alkaliionen. Die Ordinaten der dargestellten Diagramme geben jeweils die relativen Lumineszenzintensitäten an.

### 2.1. Figur D

Die Fig. D zeigt die relative Lumineszenzintensität des erfindungsgemäßen Monoaza-Kronenethers C9 in wässeriger Lösung (2x10⁻⁵ mol/l; 30 mmol/l Tris/HCl-Puffer; CO₂-frei; pH: 7,4; 37°C) in Abhängigkeit von der Wellenlänge (nm; Abszisse) bei den Na-Konzentrationen: 0, 50, 100, 150 und 200 mmol/l.

Die Anregungsspektren (links) und die Emissionsspektren (rechts) wurden mittels eines handelsüblichen Spektrofluorometers bestimmt.

### 2.2 Figur E

Die Fig. E zeigt die relative Lumineszenzintensität des erfindungsgemäßen Monoaza-Kronenethers C9, der auf Aminocellulose kovalent immobilisiert war.

Die Immobilisierung auf Cellulosefasern (aminomodifiziert) wurde wie folgt vorgenommen:

0,03 mmol des Kronenethers C9, 0,06 g (0,3 mmol) N,N-Dicyclohexyl-1,3-carbodiimid, 0,4 g (0,3 mmol) N-Hydroxysuccinimid und 5 g aktivierte Cellulose (hergestellt gemäß SU-A - 1,028,677, CA 99:177723h) wurden in 2 ml Dimethylformamid 20 Stunden suspendiert. Anschließend wurde die Cellulose abfiltriert, 5 mal mit 5 ml Dimethylformamid, 5 ml Wasser, 2 mal mit 5 ml 0,2 n HCl, 5 ml Wasser, 2 mal mit 5 ml 0,2 n NaOH, 10 mal mit 5 ml Wasser, 2 mal mit 5 ml Aceton und 2 mal mit 5 ml Ether gewaschen, und 16 Stunden bei Raumtemperatur getrocknet. Anschließend wurde die Cellulose gesiebt (25 µm).

Sensorscheiben wurden wie folgt hergestellt:

0,25 g gesiebte (25 µm) Aminocellulosefasern mit immobilisiertem Kronenether C9 wurden in 4,75 g 10% Hydrogel D4 (Tyndale Plains-Hunter LTD. Ringoes, NJ 08551) in 90% Ethanol-Wasser 16 Stunden suspendiert. Die erhaltene homogene Dispersion wurde auf eine Polyesterfolie (Melinex-Folie, ICI America) bis zu einer Trockendichte von 10 µm aufgetragen. Diese Folie wurde mit 3% Aktivkohle in 10% D4-Hydrogel bis zu einer Trockendichte von 5 µm überschichtet, worauf eine kleine Scheibe mit einem Durchmesser von 2,5 cm herausgeschnitten wurde. Diese Scheibe wurde zur Aktivierung mindestens 16 Stunden im Puffer gelassen.

Eine Methode zum Schneiden und Messen von Sensorscheiben wurde von M.J.P. Leiner und P. Hartmann in Sensors and Actuators B, 11 (1993), 281-289 ("Theory and practice in optical pH sensing") beschrieben.

Die erhaltene Sensorscheibe wurde in der in der Figur I schematisch dargestellten Meßanordnung verwendet.

In der Figur I steht das Bezugszeichen S für einen Abschnitt der Sensorscheibe. Die an den Cellulosefasern immobilisierte Verbindung ist mit I bezeichnet und liegt im Hydrogel (Schicht M) vor. Diese Schicht M ist ionenpermeabel und wird von einem für die Anregungs- und Meßstrahlung durchlässigen Träger T, der eine transparente Folie ist, getragen.

Die Verbindung I kann erfindungsgemäß an die ionenpermeable Matrix direkt kovalent gebunden sein oder in der Matrix physikalisch gelöst vorliegen.

Zur Messung wurde die Sensorscheibe in eine lichtundurchlässige, thermostatisierte Durchflußzelle eingebracht und mit Proben P, die unterschiedliche Natriumionenkonzentrationen aufwiesen, in Kontakt gebracht.

Die optische Meßeinrichtung bestand aus einer blauen LED als Lichtquelle A, einer Photodiode M als Detektor, optischen Filtern A und F zur Auswahl der Wellenlängen, einer faseroptischen Anordnung zur Leitung des Anregungslichtes in die Polymerschicht M und zur Leitung des Emissionslichtes zum Photodetektor M, sowie einer Einrichtung zur elektronischen Signalverarbeitung (nicht dargestellt). Anregungsseitig wurde ein Interferenzfilter (Peak-Transmission bei 480 nm) und emissionsseitig ein 520 nm cut-off Kantenfilter verwendet.

Die Figur E zeigt die relative Lumineszenzintensität (Ordinate) in Abhängigkeit von der Natriumionenkonzentration (10, 50, 100, 124, 144, 164, 184, 300, 500 und 1000 mmol/1; logarithmische Skala).

Das Meßmedium war 30 mmol/l Tris/HCl-Puffer, CO₂-frei; pH: 7,4; 37°C.

### Figur F

Die Figur F wurde analog Figur E erhalten, wobei jedoch anstelle des Monoaza-Kronenethers C9 der Monoaza-Kronenether C10 verwendet wurde.

### Figur G

Die Fig. G zeigt die relative Lumineszenzintensität des erfindungsgemäßen Monoaza-Kronenethers D5 in wässeriger Lösung (2x10⁻⁵ mol/l; 30 mmol/l Tris/HCl-Puffer; CO₂-frei; pH: 7,4; 37°C) in Abhängigkeit von der Wellenlänge (nm; Abszisse) bei den Na-Konzentrationen: 0, 100 und 200 mmol/l.

### Figur H

Die Figur H wurde analog Figur E erhalten, wobei jedoch anstelle des Monoaza-Kronenethers C9 der Monoaza-Kronenether D5 verwendet wurde und die Natriumionen-Konzentrationen 1, 50, 100, 138, 182 und 300 mmol/l betrugen.

## Patentansprüche

1. Verfahren zur Bestimmung eines Alkaliions in einer Probe, wobei das Alkaliion mit einer Verbindung, die einen luminophoren Rest und einen ionophoren Rest aufweist, in Kontakt gebracht wird, welcher ionophore Rest mit dem in der Probe enthaltenen Alkaliion reagiert, wobei der luminophore Rest seine Lumineszenzeigenschaften ändert, wonach die Lumineszenz gemessen und mit dem Messergebnis das Alkaliion bestimmt wird, **dadurch gekennzeichnet, dass** als Verbindung ein Monoaza-Kronenether der allgemeinen Formel I 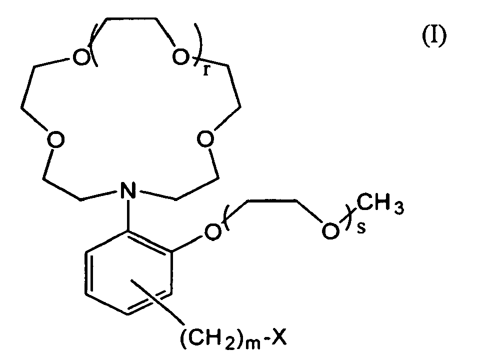 worin X der luminophore Rest ist, m die Zahl 1 oder 2 ist, und r und s unabhängig voneinander die Zahlen 0, 1 oder 2 bedeuten, eingesetzt wird.

2. Verfahren zur Bestimmung eines Alkaliions in einer Probe, wobei das Alkaliion mit einer Verbindung, die einen luminophoren Rest und einen ionophoren Rest aufweist, in Kontakt gebracht wird, welcher ionophore Rest mit dem in der Probe enthaltenen Alkaliion reagiert, wobei der luminophore Rest seine Lumineszenzeigenschaften ändert, wonach die Lumineszenz gemessen und mit dem Messergebnis das Alkaliion bestimmt wird, **dadurch gekennzeichnet, dass** als Verbindung ein Monoaza-Kronenether der allgemeinen Formel I, worin X der luminophore Rest ist, m = 0, wobei der ionophore Rest und der luminophore Rest durch Verdrehung der Ebene des luminophoren Restes zur Ebene des Benzolringes elektronisch entkoppelt sind, und r und s unabhängig voneinander die Zahlen 0, 1 oder 2 bedeuten, eingesetzt wird.

3. Verfahren nach Anspruch 1 oder 2, **dadurch gekennzeichnet, dass** zur Bestimmung von Natriumionen ein Monoaza-Kronenether der allgemeinen Formel I eingesetzt wird, in welcher r und s die Zahlen 1 bzw. 0 bedeuten.

4. Verfahren nach Anspruch 1oder 2, **dadurch gekennzeichnet, dass** zur Bestimmung von Kaliumionen ein Monoaza-Kronenether der allgemeinen Formel I eingesetzt wird, in welcher r und s die Zahlen 2 bzw. 1 bedeuten.

5. Verfahren nach einem der Ansprüche 1 bis 4, **dadurch gekennzeichnet, dass** der luminophore Rest X in der allgemeinen Formel I
- ein Amino-Naphthalimid-Rest der allgemeinen Formel II 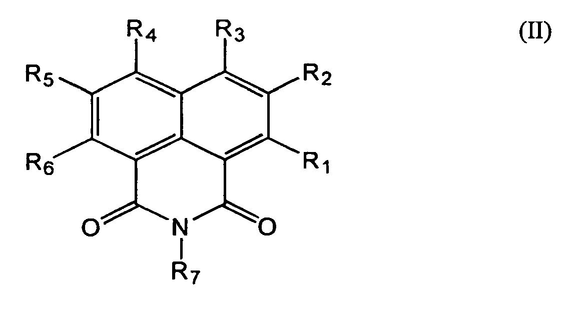 ist, in welcher eine von R₁, R₂, R₃, R₄, R₅ und R₆ eine Gruppe -NH- ist, über welche X an die Gruppe -(CH₂)ₘ- der Verbindung mit der allgemeinen Formel I gebunden ist, und die anderen und R₇ jeweils voneinander unabhängig Wasserstoff, eine lipophile oder hydrophile Gruppe oder eine reaktive Gruppe zur Kopplung an ein Polymer sind,
oder
- ein Xanthenon-Rest der allgemeinen Formel III 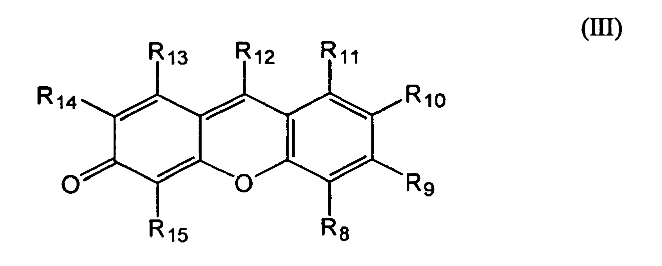 ist, in welcher eine von R₈, R₉, R₁₀, R₁₁, R₁₂, R₁₃, R₁₄ und R₁₅ für eine chemische Bindung steht, über welche X direkt (m=0) an den ionophoren Rest der Verbindung mit der allgemeinen Formel I gebunden ist, und die übrigen für -OH, OR₁₆, worin R₁₆ eine hydrophile oder eine lipophile Gruppe ist, -O-R₁₇-G, worin R₁₇ eine hydrophile oder eine lipophile Gruppe und G eine reaktive Gruppe zur Kopplung an ein Polymer ist, oder -(CH₂)ₙ-COOH, worin n eine ganze Zahl zwischen 0 und 17 ist, stehen.

6. Monoaza-Kronenether der allgemeinen Formel I 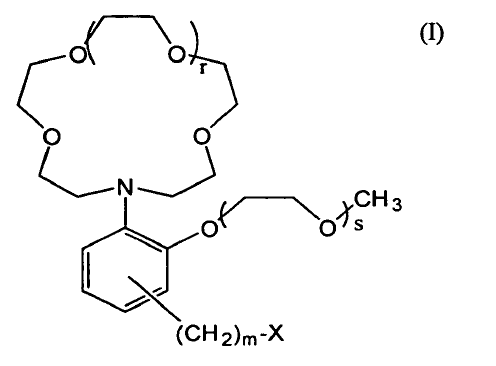 worin X der luminophore Rest ist, m die Zahl 1 oder 2 ist, und r und s unabhängig voneinander die Zahlen 0, 1 oder 2 bedeuten.

7. Monoaza-Kronenether der allgemeinen Formel I, worin X der luminophore Rest ist, m = 0, wobei der ionophore Rest und der luminophore Rest durch Verdrehung der Ebene des luminophoren Restes zur Ebene des Benzolringes elektronisch entkoppelt sind, und r und s unabhängig voneinander die Zahlen 0, 1 oder 2 bedeuten.

## Claims

1. A process for the determination of an alkali ion in a sample, wherein the alkali ion is contacted with a compound having a luminophoric moiety and an ionophoric moiety, with the ionophoric moiety reacting with the alkali ion contained in the sample, whereby the luminophoric moiety changes its luminescence properties, whereupon luminescence is measured and, via the measuring result, the alkali ion is determined, **characterized in that** a monoaza crown ether of general formula I 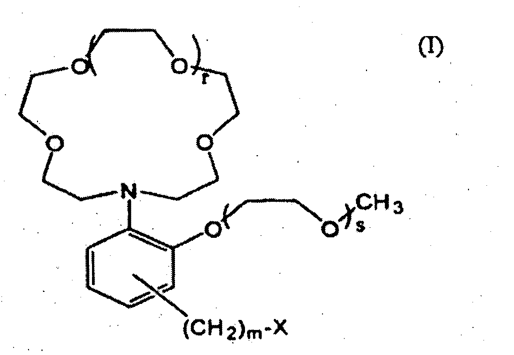 is used as the compound, wherein X is the luminophoric moiety, m is the number 1 or 2, and r and s, independently of each other, represent the numbers 0, 1 or 2.

2. A process for the determination of an alkali ion in a sample, wherein the alkali ion is contacted with a compound having a luminophoric moiety and an ionophoric moiety, with the ionophoric moiety reacting with the alkali ion contained in the sample, whereby the luminophoric moiety changes its luminescence properties, whereupon luminescence is measured and, via the measuring result, the alkali ion is determined, **characterized in that** a monoaza crown ether of general formula I is used as the compound, wherein X is the luminophoric moiety, m=0, wherein the ionophoric moiety and the luminophoric moiety are electronically decoupled by a distortion of the plane of the luminophoric moiety relative to the plane of the benzene ring, and r and s, independently of each other, represent the numbers 0, 1 or 2.

3. A process according to claim 1 or 2, **characterized in that** a monoaza crown ether of general formula I, wherein r and s represent the numbers 1 and 0, respectively, is used for the determination of sodium ions.

4. A process according to claim 1 or 2, **characterized in that** a monoaza crown ether of general formula I, wherein r and s represent the numbers 2 and 1, respectively, is used for the determination of potassium ions.

5. A process according to any of claims 1 to 4, **characterized in that** the luminophoric moiety X in general formula I is
- an amino naphthalimide moiety of general formula II 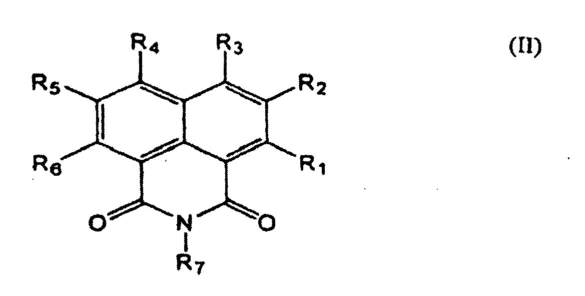
wherein one of R₁, R₂, R₃, R₄, R₅ and R₆ is a group -NH- through which X is bound to the group -(CH₂)ₘ- of the compound having the general formula I, and the others and R₇ each are, independently of each other, hydrogen, a lipophilic or hydrophilic group or a reactive group for coupling to a polymer,
or
- a xanthenone moiety of general formula III 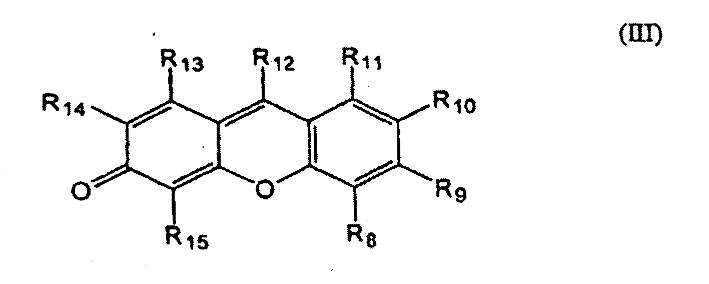
wherein one of R₈, R₉, R₁₀, R₁₁, R₁₂, R₁₃, R₁₄ and R₁₅ stands for a chemical bond through which X is directly (m=0) bound to the ionophoric moiety of the compound having the general formula I, and the remaining ones stand for -OH, OR₁₆, wherein R₁₆ is a hydrophilic or lipophilic group, -O-R₁₇-G, wherein R₁₇ is a hydrophilic or lipophilic group and G is a reactive group for coupling to a polymer, or -(CH₂)ₙ-COOH, wherein n is an integer between 0 and 17.

6. A monoaza crown ether of general formula I 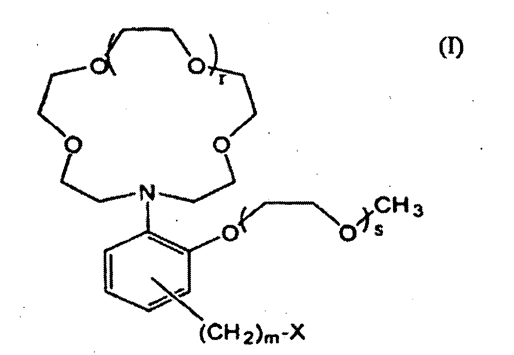 wherein X is the luminophoric moiety, m is the number 1 or 2, and r and s, independently of each other, represent the numbers 0, 1 or 2.

7. A monoaza crown ether of general formula I, wherein X is the luminophoric moiety, m=0, wherein the ionophoric moiety and the luminophoric moiety are electronically decoupled by a distortion of the plane of the luminophoric moiety relative to the plane of the benzene ring, and r and s, independently of each other, represent the numbers 0, 1 or 2.

## Revendications

1. Procédé de dosage d'un ion alcalin dans un échantillon, dans lequel on met l'ion alcalin en contact avec un composé qui présente un reste luminophore et un reste ionophore, lequel reste ionophore réagit avec l'ion alcalin contenu dans l'échantillon, ce qui entraîne la modification des propriétés de luminescence du reste luminophore, après quoi on mesure la luminescence et l'on détermine la quantité de l'ion alcalin à partir du résultat de la mesure, **caractérisé en ce que** l'on utilise comme composé un monoaza-éther couronne de formule générale I 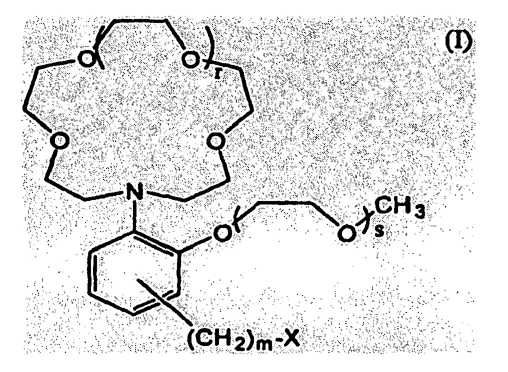 dans laquelle X est le reste luminophore, m est le nombre 1 ou 2, et r et s représentent indépendamment l'un de l'autre les nombres 0, 1 ou 2.

2. Procédé de dosage d'un ion alcalin dans un échantillon, dans lequel on met l'ion alcalin en contact avec un composé qui présente un reste luminophore et un reste ionophore, lequel reste ionophore réagit avec l'ion alcalin contenu dans l'échantillon, ce qui entraîne la modification des propriétés de luminescence du reste luminophore, après quoi on mesure la luminescence et l'on détermine la quantité de l'ion alcalin à partir du résultat de la mesure, **caractérisé en ce que** l'on utilise comme composé un monoaza-éther couronne de formule générale I dans laquelle X est le reste luminophore, m = 0, le reste ionophore et le reste luminophore étant électroniquement découplés par rotation du plan du reste luminophore par rapport au plan du cycle benzène, et r et s représentent indépendamment l'un de l'autre les nombres 0, 1 ou 2.

3. Procédé selon la revendication 1 ou 2, **caractérisé en ce que**, pour doser les ions sodium, on utilise un monoaza-éther couronne de formule générale I dans laquelle r et s représentent respectivement les nombres 1 et 0.

4. Procédé selon la revendication 1 ou 2, **caractérisé en ce que**, pour doser les ions potassium, on utilise un monoaza-éther couronne de formule générale I dans laquelle r et s représentent respectivement les nombres 2 et 1.

5. Procédé selon l'une des revendications 1 à 4, **caractérisé en ce que** le reste luminophore X de la formule générale I est
- un reste d'aminonaphtalimide de formule générale 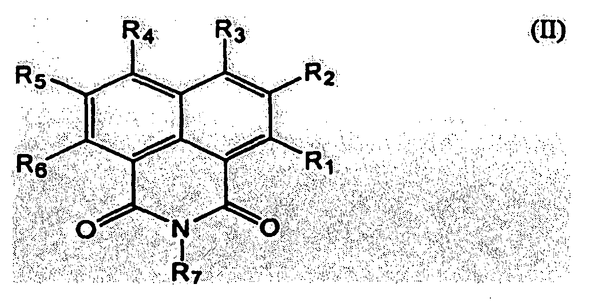 dans laquelle l'un des R₁, R₂, R₃, R₄, R₅ et R₆ est un groupe -NH- par l'intermédiaire duquel X est lié au groupe -(CH₂)ₘ- du composé de formule générale I, et les autres et R₇ sont chacun, indépendamment les uns des autres, un atome d'hydrogène, un groupe lipophile ou hydrophile
ou un groupe réactif pour le couplage à un polymère,
ou
- un reste de xanthénone de formule générale III 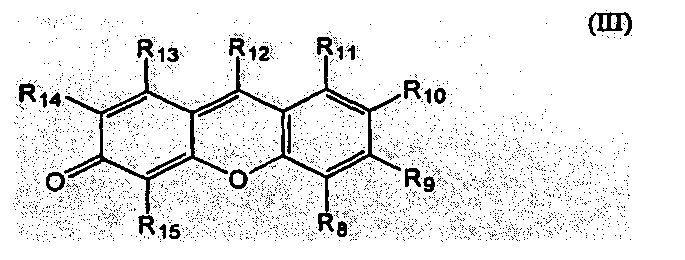
dans laquelle l'un des restes R₈, R₉, R₁₀, R₁₁, R₁₂, R₁₃, R₁₄ et R₁₅ représente une liaison chimique par l'intermédiaire de laquelle X est lié directement (m = 0) au reste ionophore du composé de formule générale I, et les autres représentent OH, OR₁₆, où R₁₆ est un groupe hydrophile ou un groupe lipophile, -O-R₁₇-G, où R₁₇ est un groupe hydrophile ou un groupe lipophile et G est un groupe réactif pour le couplage à un polymère, ou -(CH₂)ₙ-COOH, où n est un nombre entier entre 0 et 17.

6. Monoaza-éther couronne de formule générale I 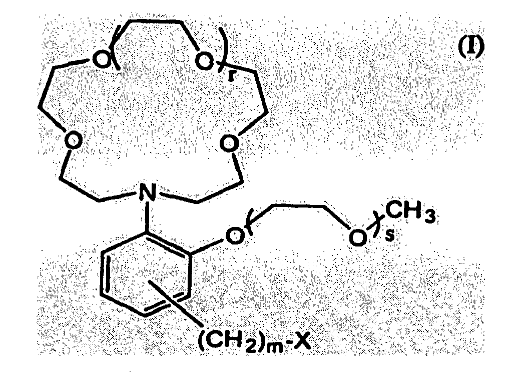 dans laquelle X est le reste luminophore, m est le nombre 1 ou 2, et r et s représentent indépendamment l'un de l'autre les nombres 0, 1 ou 2.

7. Monoaza-éther couronne de formule générale I, dans laquelle X est le reste luminophore, m = 0, le reste ionophore et le reste luminophore étant électroniquement découplés par rotation du plan du reste luminophore par rapport au plan du cycle benzène, et r et s représentent indépendamment l'un de l'autre les nombres 0, 1 ou 2.
